# EUROPEAN PATENT SPECIFICATION

(11) **EP 3 891 659 B1**
(45) Date of publication and mention of the grant of the patent: **29.01.2025**
(21) Application number: 19892196.7
(22) Date of filing: 09.12.2019
(51) Int. Cl.: A61M 16/06, A61M 16/00, G06V 20/60

(54) **INTELLIGENT SETUP AND RECOMMENDATION SYSTEM FOR SLEEP APNEA DEVICE**
INTELLIGENTE EINRICHTUNG UND EMPFEHLUNGSSYSTEM FÜR SCHLAFAPNOE
SYSTÈME DE CONFIGURATION ET DE RECOMMANDATION INTELLIGENT POUR DISPOSITIF D'APNÉE DU SOMMEIL

(30) Priority: 07.12.2018 US 201862777044 P
(43) Date of publication of application: 13.10.2021
(73) Proprietor: ResMed Inc., San Diego CA 92123 (US)
(72) Inventor: SODHI, Rajwant, San Diego, California 92123 (US); NATHWANI, Rehana, San Diego, California 92123 (US); KENNEDY, Colin Bradley, San Diego, California 92123 (US)
(74) Representative: Mathys & Squire
(86) International application number: PCT/US2019/065295
(87) International publication number: WO 2020/118311

(56) References cited:
- US-A1- 2008 060 652
- US-A1- 2015 306 330
- US-A1- 2016 092 645
- US-A1- 2016 193 437
- US-A1- 2017 296 764
- ANONYMOUS: "Sleep Apnea Support", 16 July 2017 (2017-07-16), pages 1 - 4, XP009528598, Retrieved from the Internet <URL:https://web.archive.org/web/20170716134838/http://www.resmed.com/us/en/consumer/support.html> [retrieved on 20200123]

## Description

### PRIORITY CLAIM

The present application claims priority from U.S. Provisional Application Serial No. 62/777,044 filed December 7, 2018 and titled "Intelligent Setup and Recommendation System For Sleep Apnea Device."

### TECHNICAL FIELD

The present disclosure relates generally to a sleep apnea system, and more specifically, to an intelligent setup and recommendation system for sleep apnea patients.

### BACKGROUND

A range of respiratory disorders exist. Certain disorders may be characterized by particular events, such as apneas, hypopneas, and hyperpneas. Obstructive Sleep Apnea (OSA), a form of Sleep Disordered Breathing (SDB), is characterized by events including occlusion or obstruction of the upper air passage during sleep. It results from a combination of an abnormally small upper airway and the normal loss of muscle tone in the region of the tongue, soft palate and posterior oropharyngeal wall during sleep. The condition causes the affected patient to stop breathing for periods typically of 30 to 120 seconds in duration, sometimes 200 to 300 times per night. It often causes excessive daytime somnolence, and it may cause cardiovascular disease and brain damage. The syndrome is a common disorder, particularly in middle aged overweight males, although a person affected may have no awareness of the problem.

Other sleep related disorders include Cheyne-Stokes Respiration (CSR), Obesity Hyperventilation Syndrome (OHS) and Chronic Obstructive Pulmonary Disease (COPD). COPD encompasses any of a group of lower airway diseases that have certain characteristics in common. These include increased resistance to air movement, extended expiratory phase of respiration, and loss of the normal elasticity of the lung. Examples of COPD are emphysema and chronic bronchitis. COPD is caused by chronic tobacco smoking (primary risk factor), occupational exposures, air pollution and genetic factors.

Continuous Positive Airway Pressure (CPAP) therapy has been used to treat Obstructive Sleep Apnea (OSA). Application of continuous positive airway pressure acts as a pneumatic splint and may prevent upper airway occlusion by pushing the soft palate and tongue forward and away from the posterior oropharyngeal wall.

Non-invasive ventilation (NIV) provides ventilatory support to a patient through the upper airways to assist the patient in taking a full breath and/or maintain adequate oxygen levels in the body by doing some or all of the work of breathing. The ventilatory support is provided via a patient interface. NIV has been used to treat CSR, OHS, COPD, and Chest Wall disorders. In some forms, the comfort and effectiveness of these therapies may be improved. Invasive ventilation (IV) provides ventilatory support to patients that are no longer able to effectively breathe themselves and may be provided using a tracheostomy tube.

A treatment system may comprise a Respiratory Pressure Therapy Device (RPT device), an air circuit, a humidifier, a patient interface, and data management. A patient interface may be used to interface respiratory equipment to its wearer, for example by providing a flow of air to an entrance to the airways. The flow of air may be provided via a mask to the nose and/or mouth, a tube to the mouth or a tracheostomy tube to the trachea of a patient. Depending upon the therapy to be applied, the patient interface may form a seal, e.g., with a region of the patient's face, to facilitate the delivery of gas at a pressure at sufficient variance with ambient pressure to effect therapy, e.g., at a positive pressure of about 10 cm H20 relative to ambient pressure. For other forms of therapy, such as the delivery of oxygen, the patient interface may not include a seal sufficient to facilitate delivery to the airways of a supply of gas at a positive pressure of about 10 cm H20. Treatment of respiratory ailments by such therapy may be voluntary, and hence patients may elect not to comply with therapy if they find devices used to provide such therapy uncomfortable, difficult to use, expensive and/or aesthetically unappealing.

CPAP therapy is highly effective to treat certain respiratory disorders, provided patients comply with therapy. Obtaining a proper patient interface and properly setting up the CPAP machine allows a patient to engage in positive pressure therapy. Patients currently must rely on paper instructions provided by the device manufacturers for setting up their device. An improper set up or configuration often frustrates patients and thus causes the improper operation of the sleep apnea device. Thus, a sleep apnea device that is not properly configured for a particular patient may result in ineffective therapy

There is a need for a system that allows for more efficient and active setup of a sleep apnea device. There is also a need to use a mobile computing device with an augmented reality interface to assist a user to setup of a sleep apnea device. There is also a need for a patient assistance device that may evaluate the operation of a sleep apnea device. Patent publication US 2017/296764 A1 (PAUL ZACHARY DEAN [US]) 19 October 2017 (2017-10-19) discloses a patient sleep therapy self management tool.

### SUMMARY

The disclosed system provides an adaptable system to size masks for use with an RPT device for better compliance for individual patients. The system collects facial data from a primary patient and also RPT use and other data from a larger population of patients to assist in the selection of an optimal mask for the primary patient.

One disclosed example is a system to provide assistance to a patient for using a respiratory therapy device and a mask for treatment of respiratory ailments. The system includes an equipment database storing data relating to a plurality of device types and a plurality of mask types. A device recognition module is operable to identify the type of the respiratory therapy device from an image of the respiratory therapy device captured by a client computing device in comparison with the data relating to the plurality of device types. A mask recognition module is operable to identify the type of the mask from an image of the mask captured by the client computing device in comparison with the data relating to the plurality of mask types. A media database includes media relating to assistance information relating to at least one of a mask type or a device type. A management server is operable to send media relating to assistance information for the identified type of mask or identified type of device to the client computing device.

Another disclosed example is A method for providing automated assistance to a patient using a respiratory therapy device connected to a mask. An image of the respiratory therapy device and an image of the mask are captured by a client computing device. The type of the respiratory therapy device is identified from an image of the respiratory therapy device captured by the client computing device in comparison with the data relating to the plurality of device types in an equipment database via a device recognition module. The type of the mask is identified from an image of the mask captured by the client computing device in comparison with the data relating to the plurality of mask types in the equipment database via a mask recognition module. Media relating to assistance information for the identified type of mask or identified type of device is sent to the client computing device via a management server.

The above summary is not intended to represent each embodiment or every aspect of the present disclosure. Rather, the foregoing summary merely provides an example of some of the novel aspects and features set forth herein. The above features and advantages, and other features and advantages of the present disclosure, will be readily apparent from the following detailed description of representative embodiments and modes for carrying out the present invention, when taken in connection with the accompanying drawings and the appended claims.

### BRIEF DESCRIPTION OF THE DRAWINGS

The disclosure will be better understood from the following description of exemplary embodiments together with reference to the accompanying drawings, in which:
FIG. 1 illustrates an example computing environment for a sleep apnea system that allows assistance for setup of a sleep apnea device;
FIG. 2 illustrates an example of a management server in the computing environment of FIG. 1;
FIG. 3A shows a respiratory pressure therapy device in accordance with one form of the present technology;
FIG. 3B is a schematic diagram of the pneumatic path of a respiratory pressure therapy device in accordance with one form of the present technology;
FIG. 3C is a schematic diagram of the electrical components of a respiratory pressure therapy device in accordance with one form of the present technology;
FIGs. 4A-4B illustrate screenshots provided by an example client application associated with an initial welcome, login, and onboarding process of the device setup;
FIGs. 5A-5B illustrate screenshots provided by the example client application in association with an unboxing and equipment identification process;
FIGs. 6A-6B illustrate screenshots provided by the example client application in association with assisting a patient with mask placement;
FIGs. 7A-7C illustrate screenshots associated with a mask leak detection process provided by the example client application;
FIG. 8 illustrates screenshots provided by the example client application in association with a setup completion process;
FIG. 9A-B illustrate screenshots provided by the example client application in association with preparing a patient for a first night of therapy with a newly setup sleep apnea device;
FIGs. 10A-10C illustrate screenshots provided by the example client application in association with obtaining feedback from the patient;
FIG. 11 illustrates an example of a mask recognition module of the example client application; and
FIG. 12 illustrates an example of a mask recommendation module of the example client application.

The present disclosure is susceptible to various modifications and alternative forms. Some representative embodiments have been shown by way of example in the drawings and will be described in detail herein.

### DETAILED DESCRIPTION OF THE ILLUSTRATED EMBODIMENTS

The present inventions can be embodied in many different forms. Representative embodiments are shown in the drawings, and will herein be described in detail. The present disclosure is an example or illustration of the principles of the present disclosure, and is not intended to limit the broad aspects of the disclosure to the embodiments illustrated. To that extent, elements and limitations that are disclosed, for example, in the Abstract, Summary, and Detailed Description sections, but not explicitly set forth in the claims, should not be incorporated into the claims, singly or collectively, by implication, inference, or otherwise. For purposes of the present detailed description, unless specifically disclaimed, the singular includes the plural and vice versa; and the word "including" means "including without limitation." Moreover, words of approximation, such as "about," "almost," "substantially," "approximately," and the like, can be used herein to mean "at," "near," or "nearly at," or "within 3-5% of," or "within acceptable manufacturing tolerances," or any logical combination thereof, for example.

The present disclosure relates to an automated assistant that assists a user or a respiratory therapy device. The assistant allows a user to confirm both the type of device and mask. The assist includes

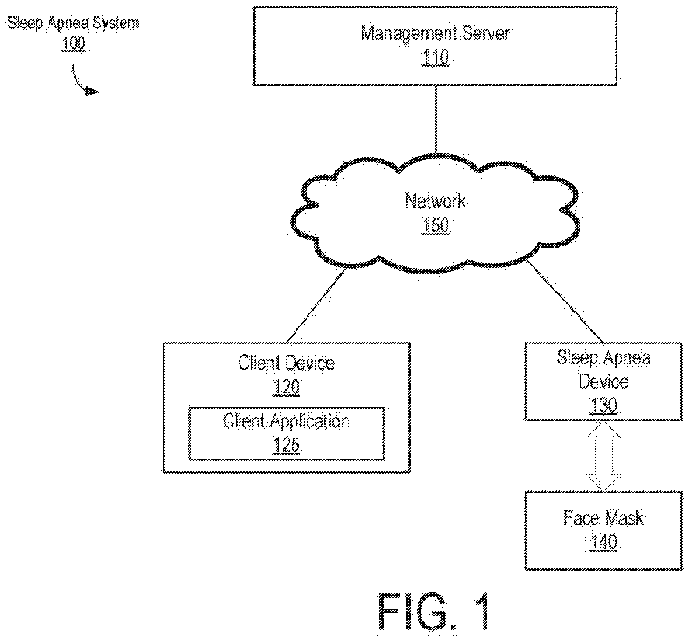

FIG. 1 illustrates an example computing environment for a sleep apnea system 100. The sleep apnea system 100 comprises a management server 110, a client computing device 120, a sleep apnea device 130, and a mask 140. Although only a single client device 120, sleep apnea device 130, and mask 140 are illustrated, an embodiment of the computing environment 100 may have hundreds or thousands of client devices 120, sleep apnea devices 130, and masks 140 managed by the management server 110.

The sleep apnea device 130 may comprise, for example, a continuous positive airway pressure (CPAP) device, an automatic positive airway pressure (APAP) device, a bilevel positive airway pressure device (BiPAP) or other device for treating sleep apnea. Such devices are generally referenced as respiratory therapy devices (RPT). The sleep apnea device 130 generally comprises a pressurized ventilator that connects to a face mask 140 via a hose. The sleep apnea device 130 applies mild air pressure through the hose and mask 140 to keep the patient's airways open during sleep. In a CPAP device, the air pressure flows to the mask 140 at a constant pressure. In a BiPAP device, the air pressure switches between two different pressure levels for inhalation and exhalation respectively. In an APAP device, the sleep apnea device 130 senses changes in breathing and adjusts the air pressure to an appropriate level based on the breathing pattern.

The sleep apnea device 130 may include a wireless or wired communication interface to connect to the management server 110 via the network 150. For example, the sleep apnea device 130 may communicate with the network 150 via a WiFi connection, a cellular connection, an Ethernet connection, or other connection. The sleep apnea device 130 may include sensors that monitor various data associated with the patient's usage and sends the data to the management server 110. For example, the sleep apnea device 130 may sense the patient's breathing rate, respiratory flow, and overall usage patterns (e.g., how often and for how long the patient uses the device 130), and reports this information to the management server 110.

The mask 140 couples to the sleep apnea device 130 via a hose and receives the pressurized air flow generated by the sleep apnea device 130. The mask 140 is designed to be worn around the patient's mouth, nose, or both. When properly sized and worn, the mask 140 provides an airtight seal around the patient's mouth, nose, or both to enable the pressurized air to flow into the patient's breathing cavities. The mask 140 may furthermore include one or more straps that wrap around the patient's head to secure the mask 140 in place. The straps may be adjustable to provide a proper fit and seal for a given patient.

The client computing device 120 comprises a network-enabled computing device such as a computer, a mobile device, or tablet that executes a client application 125. The client application 125 provides a user interface on a display that enables the patient to provide various information to the management server 110 and view various information from the management server 110 relating to the patient's treatment for respiratory ailments using the sleep apnea device 130. For example, the client application 125 may enable the patient to set up a patient profile that includes various characteristics of the patient (e.g., gender, weight, height, sleep habits, etc.) and link the profile to the sleep apnea device 130. The client application 125 may furthermore provide various interfaces on the display to assist the patient through initial unboxing, setup, and usage of the sleep apnea device 130. Additionally, the client application 125 may enable the patient to access various usage data associated with the patient's usage of the sleep apnea device 130. Furthermore, the client application 125 may provide alerts and recommendations from the management server 110 relating to the patient's treatment such as, for example, alerting the patient when it is time to replace the face mask 140, alerting the patient when the patient is not following the recommended usage amount, alerting the patient when a malfunction is detected with the sleep apnea device 130 or mask 140, etc. The client application 125 may also enable the patient to provide feedback to the management server 110 relating to the patient's experience with the treatment. While not shown in FIG. 1, client device 120 may be configured to communicate directly with sleep apnea device 130 via a network connection such as, for example, Bluetooth, WiFi, cellular, and/or other communication mechanisms.

The management server 110 comprises a computer or set of computers providing various management and control functions associated with one or more sleep apnea devices 130. For example, the management server 110 may obtain data associated with patients, their devices 130, and masks 140, and generate content to assist the patient in setting up and using device 130 and mask 140. Furthermore, the management server 110 may collect various usage data associated with sleep apnea treatment in order to generate recommendations to patients that are tailored to their particular characteristics.

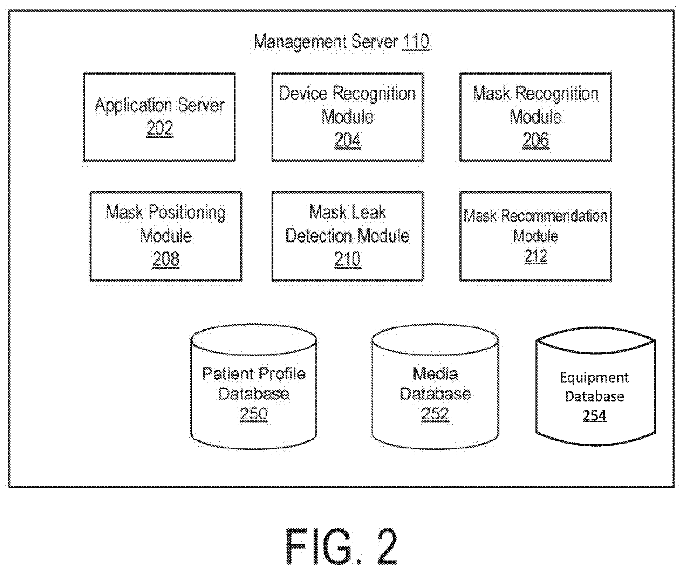

FIG. 2 illustrates an example embodiment of a management server 110. The management server 110 comprises an application server 202, a device recognition module 204, a mask recognition module 206, a mask positioning module 208, a mask leak detection module 210, a mask recommendation module 212, a patient profile database 250, a media database 252, and an equipment database 254. In alternative embodiments, the management server 110 may include additional or different modules. In an embodiment, each of the modules may include computer-executable instructions stored to a non-transitory storage medium that when executed by a processor cause the processor to carry out functions attributed to the modules as described herein.

The application server 202 provides an interface between the management server 110 and the client application 125. The application server 202 exchange data and control information with the client application 125 to cause the client application 125 to carry out various functions described herein. For example, the application server 202 may obtain profile information from a patient when the patient first opens the client application 125 and may store the profile information to the patient profile database 250. The application server 202 may furthermore cause the client application 125 to present various user interface screens to guide the user through an onboarding and setup process. Further still, the application server 202 may obtain various sensor data, usage information, and surveyed answers associated with the patient throughout the patient's treatment. The application server 202 may also provide the patient with access to various information to assist the patient with carrying out treatment for respiratory ailments such as sleep apnea.

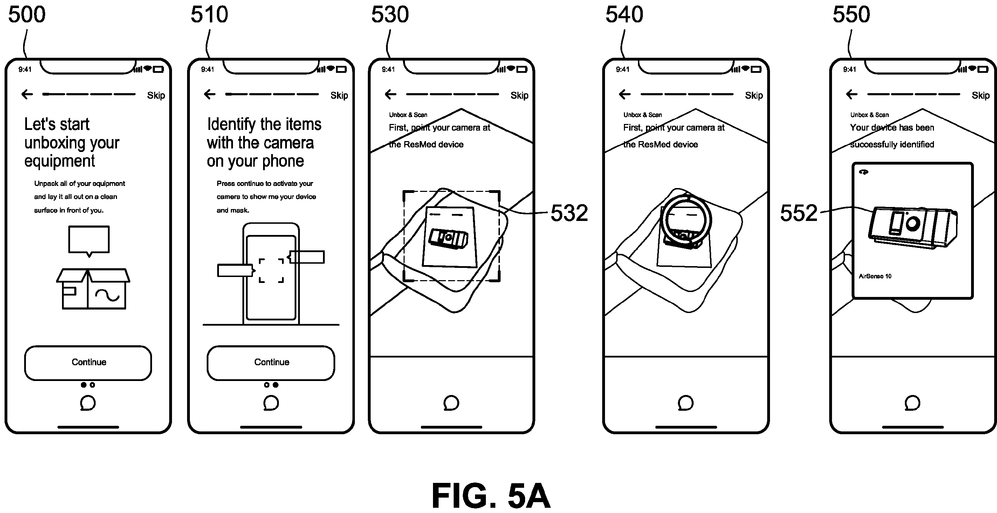

The device recognition module 204 comprises a machine-learning model that enables automatic detection of a particular model of sleep apnea device 130 based on one or more images of the sleep apnea device 130 captured by the patient via the client device 120. In an embodiment, the machine-learning model is based on learned correlations between image features and device models from different device type data stored in the equipment database 254. For example, to generate the machine-learning model, a large number of images may be captured of a device 130 of each different model from a variety of angles, lighting conditions, and camera specifications. Features may be extracted from the images, and a learning algorithm may learn which features most strongly correlate to the device model. During operation, the device recognition module 204 may receive an image of a sleep apnea device 130 (or features extracted from an image) and predict the device model based on the machine-learning model. An example user interface associated with the device recognition module 204 is described in further detail below with respect to FIG. 5A.

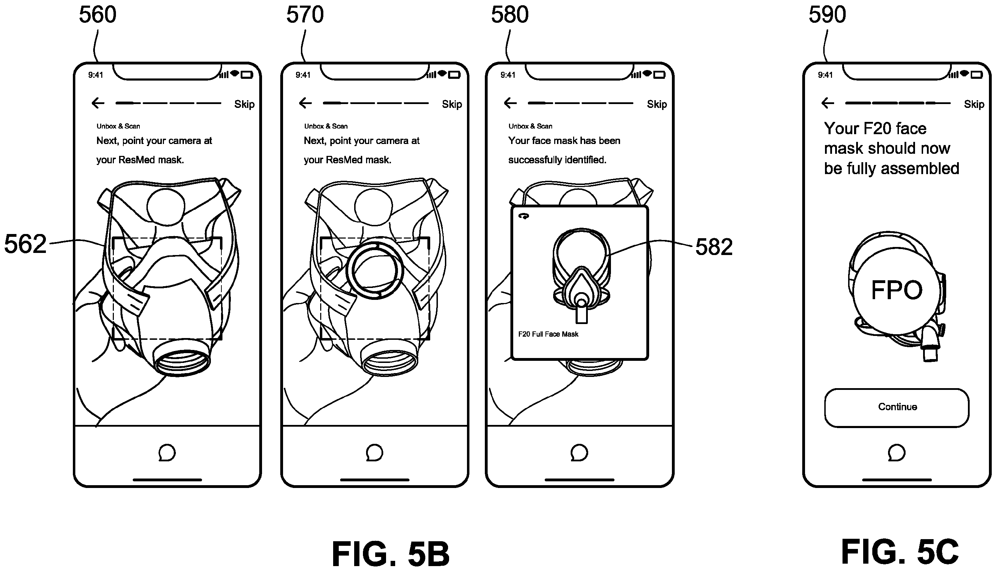

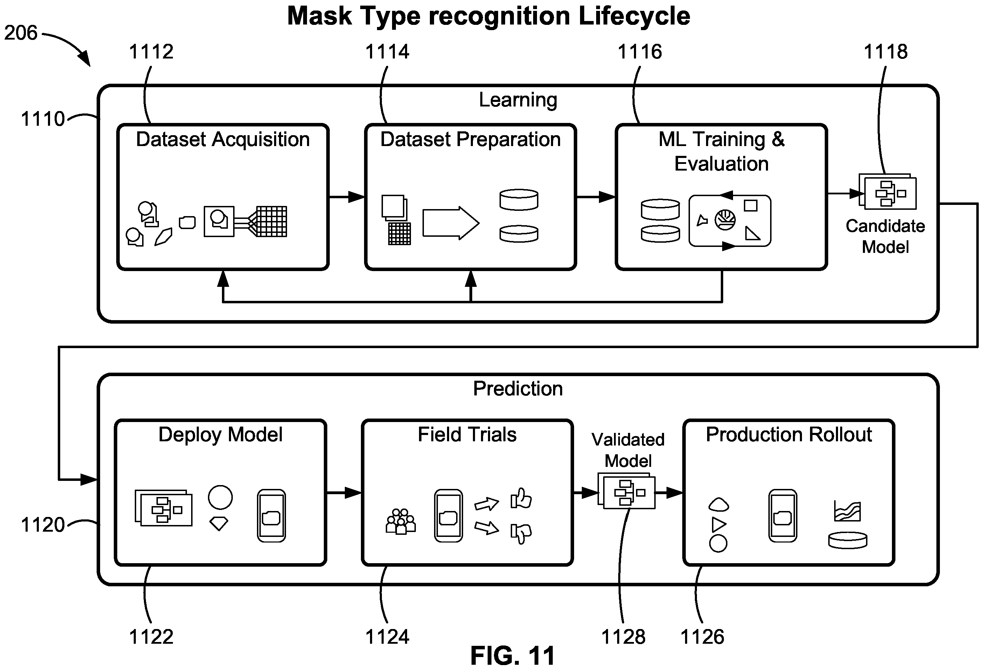

The mask recognition module 206 comprises a machine-learning model that enables automatic detection of a particular model of a mask 140 based on one or more images of the mask 140 captured by the patient via the client device 120. In an embodiment, the machine-learning model is based on learned correlations between image features and mask models. For example, to generate the machine-learning model, a large number of images may be captured of a mask 140 of each different model from a variety of angles, lighting conditions, and camera specifications. Features may be extracted from the images, and a learning algorithm may learn which features most strongly correlate to the mask model. According to some embodiments, the learning algorithm may use mask CAD files stored in the equipment database 254 as inputs in analyzing features and correlating such features to a particular mask model. During operation, the mask recognition module 206 may receive an image of a mask 140 (or features extracted from an image) and predict the mask model based on the machine-learning model. An example user interface associated with the mask recognition module 206 is described in further detail below with respect to FIG. 5B. A more detailed description of the mask recognition module 206 is furthermore described below with respect to FIG. 11.

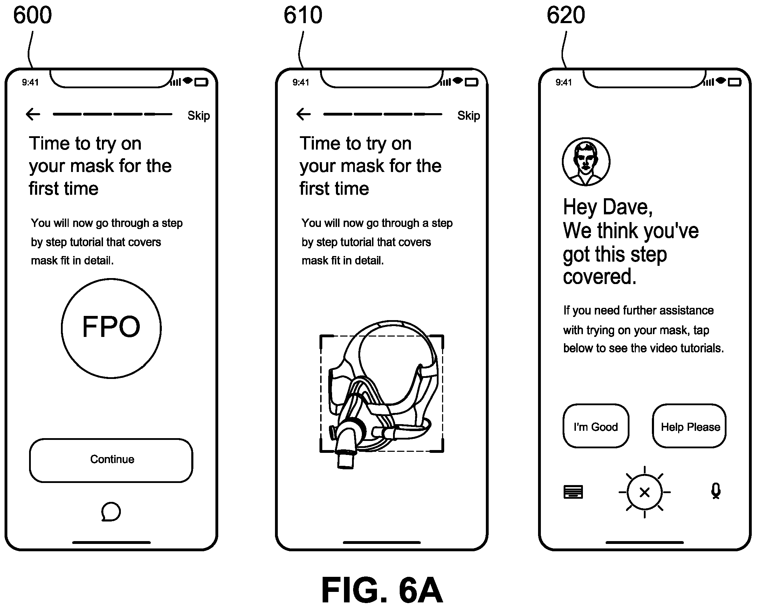

The mask positioning module 208 provides an augmented reality interface to assist a patient with properly placing the mask 140 on the patient's face. The mask positioning module 208 receives an input video stream of a portrait of the patient that may be captured via a camera of the client device 120. The mask positioning module 208 performs a facial analysis to identify and track locations of particular facial landmarks. The mask positioning module 208 then overlays a mask placement image (e.g., an image of a mask 140 or an outline of a mask 140) on the received image frames at the appropriate position aligned to the detected facial landmarks. The image frames are sent to the client application 125 as an augmented reality view of the patient's face with the overlaid mask placement image. Based on the augmented reality view, the patient may align the mask 140 to the mask placement image indicative of the proper mask placement. An example user interface associated with the mask positioning module 208 is described in further detail below with respect to FIGs. 6A-6C.

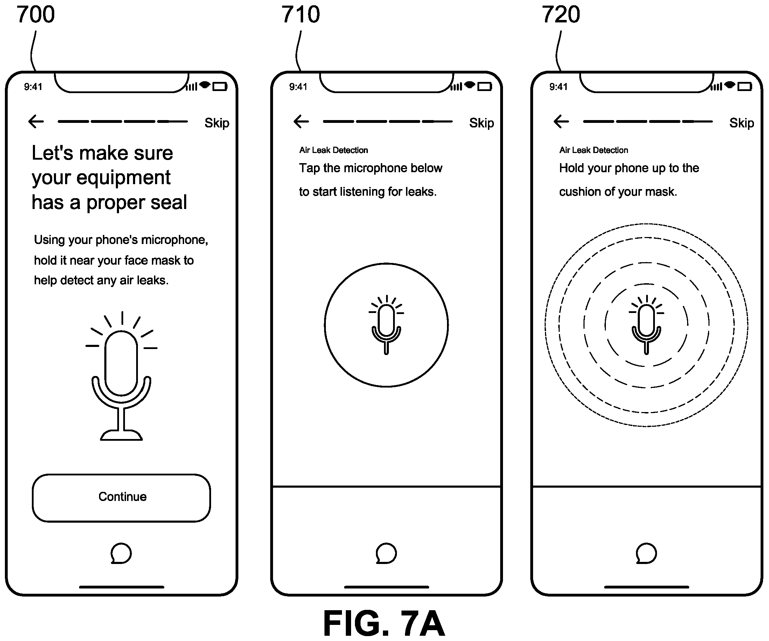

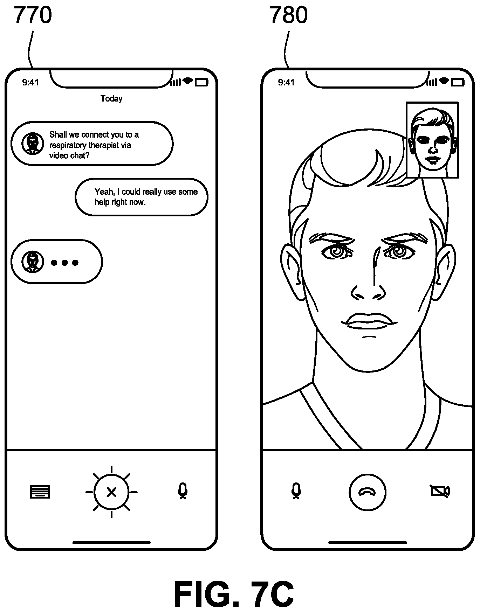

The mask leak detection module 210 detects whether a mask 140 is properly sealed around a patient's face based on captured audio of the patient's breathing while the sleep apnea device is delivering therapy/pressure to the patient. For example, after the patient places the mask 140, the client device 110 may be configured to capture audio (using a microphone of the client device 110) of the patient's breathing. The captured audio (or features derived from the audio) may be sent to the management server 110. The mask leak detection module 210 detects the presence or absence of audio features indicative of a leak. In an embodiment, the mask leak detection module 210 may apply a machine-learning model that learns correlations between audio features extracted from audio of breathing when a leak is present. The mask positioning module 208 may also be configured to capture information from the client device 110 on how the client device 110 is positioned. Such information may then be used to aid in determining the location on the patient's face that leak is occurring. The leak detection result may be provided to the client application 125. An example user interface associated with the mask leak detection module 210 is described in further detail below with respect to FIGs. 7A-7C.

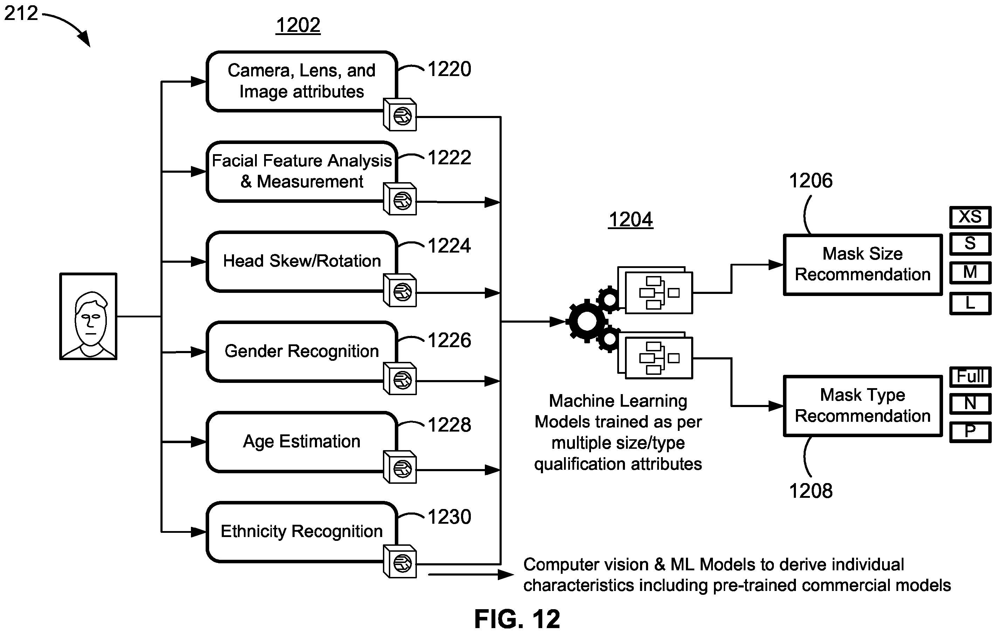

The mask recommendation module 212 generates a patient-specific recommendation for a type and size of mask 140 based an image of the patient and various characteristics of the patient. For example, the mask recommendation module 212 may obtain the image of the patient (or features derived from the image) from the client device 110 and perform a facial analysis, which may be based in part on machine-learning, to identify a mask size that is predicted to suit the patient's face and a type of mask 140 that is predicted to best fit the patient's face. In an embodiment, the mask recommendation module 212 may apply a machined-learning model to learn correlations between facial features of the patient that can be extracted from the image and the mask size and type. An example of a mask recommendation module 212 is described in further detail below with respect to FIG. 12.

The patient profile database 250 stores various information about a patient. For example, the patient profile database 250 may store physical information such as age, gender, height, weight, etc., medical history, and various preferences. The patient profile database 250 may be updated during ongoing therapy to store various usage data associated with the patient's treatment and patient-provided feedback.

The media database 252 stores various images, videos, animations, or other media helpful to assisting the patient with setting up and configuring the sleep apnea system 100. For example, the media database 252 may include images or videos showing a patient how to set up, turn on, and use the sleep apnea device 130, how to size, adjust, and place the mask 140, or other help content to reduce the patient's learning curve in beginning sleep apnea therapy.

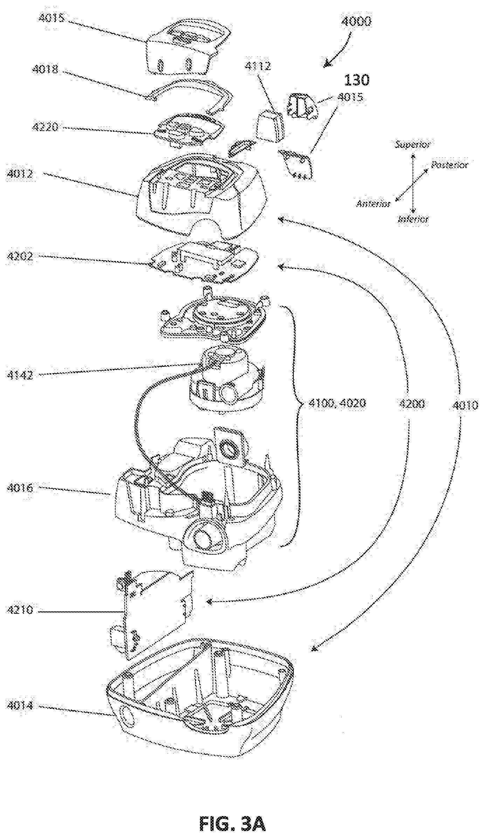

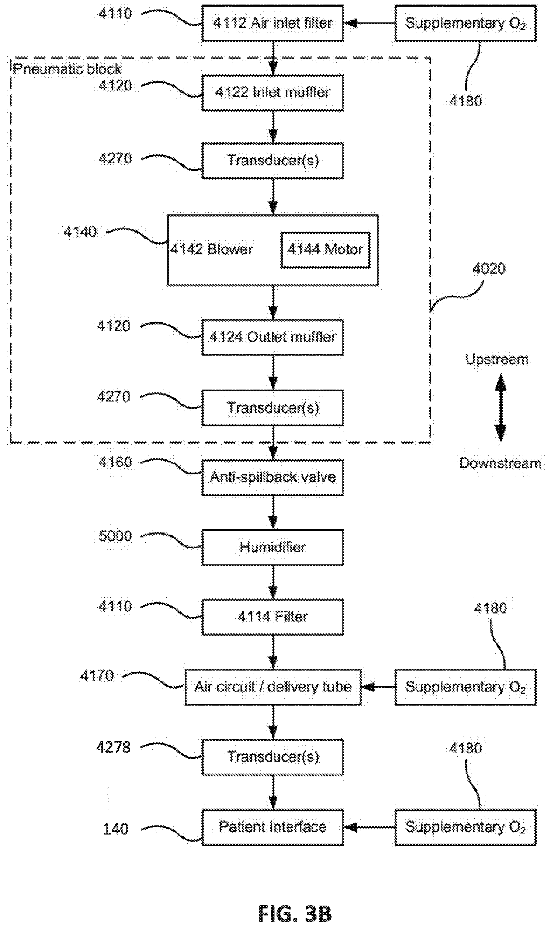

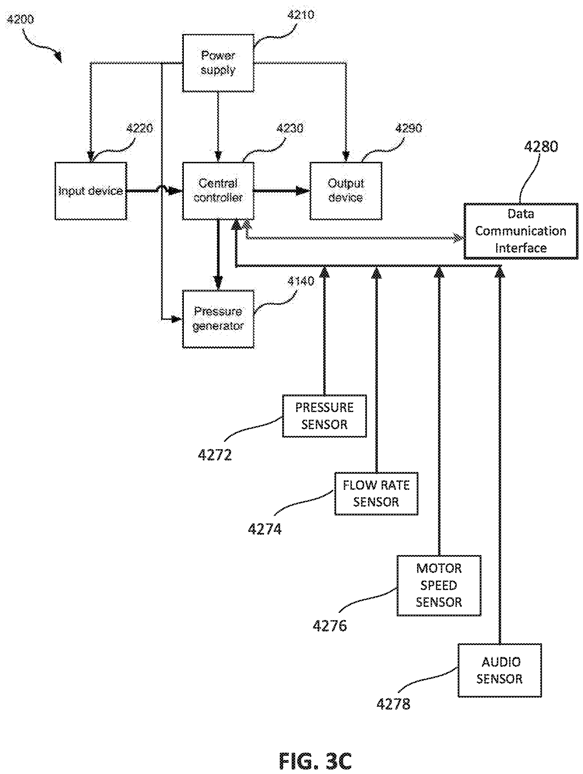

FIG. 3A shows an exploded view of the components of an example RPT device such as the sleep apnea device 130 in accordance with one aspect of the present technology comprises mechanical, pneumatic, and/or electrical components and is configured to execute one or more algorithms, such as any of the methods, in whole or in part, described herein. FIG. 3B shows a block diagram of the example RPT device 130. FIG. 3C shows a block diagram of the electrical control components of the example RPT device 130. The directions of upstream and downstream are indicated with reference to the blower and the patient interface. The blower is defined to be upstream of the patient interface and the patient interface is defined to be downstream of the blower, regardless of the actual flow direction at any particular moment. Items which are located within the pneumatic path between the blower and the patient interface are downstream of the blower and upstream of the patient interface. The RPT device 130 may be configured to generate a flow of air for delivery to a patient's airways, such as to treat one or more of the respiratory conditions.

The RPT device 130 may have an external housing 4010, formed in two parts, an upper portion 4012 and a lower portion 4014. Furthermore, the external housing 4010 may include one or more panel(s) 4015. The RPT device 130 comprises a chassis 4016 that supports one or more internal components of the RPT device 130. The RPT device 130 may include a handle 4018.

The pneumatic path of the RPT device 130 may comprise one or more air path items, e.g., an inlet air filter 4112, an inlet muffler 4122, a pressure generator 4140 capable of supplying air at positive pressure (e.g., a blower 4142), an outlet muffler 4124 and one or more transducers 4270, such as a pressure sensor 4272, a flow rate sensor 4274, and a motor speed sensor 4276.

One or more of the air path items may be located within a removable unitary structure which will be referred to as a pneumatic block 4020. The pneumatic block 4020 may be located within the external housing 4010. In one form a pneumatic block 4020 is supported by, or formed as part of the chassis 4016.

The RPT device 130 may have an electrical power supply 4210, one or more input devices 4220, a central controller 4230, a pressure generator 4140, a data communication interface 4280, and one or more output devices 4290. A separate controller may be provided for the therapy device. Electrical components 4200 may be mounted on a single Printed Circuit Board Assembly (PCBA) 4202. In an alternative form, the RPT device 130 may include more than one PCBA 4202. Other components such as the one or more protection circuits 4250, transducers 4270, the data communication interface 4280, and storage devices may also be mounted on the PCBA 4202.

An RPT device may comprise one or more of the following components in an integral unit. In an alternative form, one or more of the following components may be located as respective separate units.

An RPT device in accordance with one form of the present technology may include an air filter 4110, or a plurality of air filters 4110. In one form, an inlet air filter 4112 is located at the beginning of the pneumatic path upstream of a pressure generator 4140. In one form, an outlet air filter 4114, for example an antibacterial filter, is located between an outlet of the pneumatic block 4020 and a patient interface such as the mask 140 in FIG. 1.

An RPT device in accordance with one form of the present technology may include a muffler 4120, or a plurality of mufflers 4120. In one form of the present technology, an inlet muffler 4122 is located in the pneumatic path upstream of a pressure generator 4140. In one form of the present technology, an outlet muffler 4124 is located in the pneumatic path between the pressure generator 4140 and a patient interface such as the mask 140 in FIG. 1.

In one form of the present technology, a pressure generator 4140 for producing a flow, or a supply, of air at positive pressure is a controllable blower 4142. For example, the blower 4142 may include a brushless DC motor 4144 with one or more impellers. The impellers may be located in a volute. The blower may be capable of delivering a supply of air, for example at a rate of up to about 120 litres/minute, at a positive pressure in a range from about 4 cm H2O to about 20 cm H2O, or in other forms up to about 30 cm H2O. The blower may be as described in any one of the following patents or patent applications U.S. Patent No. 7,866,944; U.S. Patent No. 8,638,014; U.S. Patent No. 8,636,479; and PCT Patent Application Publication No. WO 2013/020167.

The pressure generator 4140 is under the control of the therapy device controller 4240. In other forms, a pressure generator 4140 may be a piston-driven pump, a pressure regulator connected to a high pressure source (e.g. compressed air reservoir), or a bellows.

An air circuit 4170 in accordance with an aspect of the present technology is a conduit or a tube constructed and arranged to allow, in use, a pressurized flow of air to travel between two components such as a humidifier 5000 and the patient interface 140. In particular, the air circuit 4170 may be in fluid communication with the outlet of the humidifier 5000 and the plenum chamber of the patient interface 140.

In one form of the present technology, an anti-spill back valve 4160 is located between the humidifier 5000 and the pneumatic block 4020. The anti-spill back valve is constructed and arranged to reduce the risk that water will flow upstream from the humidifier 5000, for example to the motor 4144.

A power supply 4210 may be located internal or external of the external housing 4010 of the RPT device 130. In one form of the present technology, power supply 4210 provides electrical power to the RPT device 130 only. In another form of the present technology, power supply 4210 provides electrical power to both RPT device 130 and humidifier 5000.

An RT system may comprise one or more transducers (sensors) 4270 configured to measure one or more of any number of parameters in relation to an RT system, its patient, and/or its environment. A transducer may be configured to produce an output signal representative of the one or more parameters that the transducer is configured to measure.

The output signal may be one or more of an electrical signal, a magnetic signal, a mechanical signal, a visual signal, an optical signal, a sound signal, or any number of others which are known in the art.

A transducer may be integrated with another component of an RPT system, where one exemplary arrangement would be the transducer being internal of an RPT device. A transducer may be substantially a 'standalone' component of an RT system, an exemplary arrangement of which would be the transducer being external to the RPT device.

A transducer may be configured to communicate its output signal to one or more components of an RT system, such as an RPT device, a local external device, or a remote external device. External transducers may be for example located on a patient interface, or in an external computing device, such as a smartphone. External transducers may be located for example on or form part of the air circuit, e.g., the patient interface.

The one or more transducers 4270 may be constructed and arranged to generate signals representing properties of air such as a flow rate, a pressure or a temperature. The air may be a flow of air from the RPT device 130 to a patient, a flow of air from the patient to the atmosphere, ambient air or any others. The signals may be representative of properties of the flow of air at a particular point, such as the flow of air in the pneumatic path between the RPT device 130 and the patient. In one form of the present technology, one or more transducers 4270 are located in a pneumatic path of the RPT device 130, such as downstream of the humidifier 5000.

In accordance with one aspect of the present technology, the one or more transducers 4270 comprises a pressure sensor located in fluid communication with the pneumatic path. An example of a suitable pressure sensor is a transducer from the HONEYWELL ASDX series. An alternative suitable pressure sensor is a transducer from the NPA Series from GENERAL ELECTRIC. In one implementation, the pressure sensor is located in the air circuit 4170 adjacent the outlet of the humidifier 5000.

A microphone pressure sensor 4278 is configured to generate a sound signal representing the variation of pressure within the air circuit 4170. The sound signal from the microphone 4278 may be received by the central controller 4230 for acoustic processing and analysis as configured by one or more of the algorithms described below. The microphone 4278 may be directly exposed to the airpath for greater sensitivity to sound, or may be encapsulated behind a thin layer of flexible membrane material. This membrane may function to protect the microphone 4278 from heat and/or humidity.

Data from the transducers 4270 such as the pressure sensor 4272, flow rate sensor 4274, motor speed sensor 4276, and microphone 4278 may be collected by central controller 4230 on a periodic basis. Such data generally relates to the operational state of the RPT device 130. In this example, the central controller 4230 encodes such data from the sensors in a proprietary data format. The data may also be coded in a standardized data format.

In one form of the present technology, an RPT device 130 includes one or more input devices 4220 in the form of buttons, switches or dials to allow a person to interact with the device. The buttons, switches or dials may be physical devices, or software devices accessible via a touch screen. The buttons, switches or dials may, in one form, be physically connected to the external housing 4010, or may, in another form, be in wireless communication with a receiver that is in electrical connection to the central controller 4230. In one form, the input device 4220 may be constructed and arranged to allow a person to select a value and/or a menu option.

In one form of the present technology, the central controller 4230 is one or a plurality of processors suitable to control an RPT device 130. Suitable processors may include an x86 INTEL processor, a processor based on ARM^{®} Cortex^{®}-M processor from ARM Holdings such as an STM32 series microcontroller from ST MICROELECTRONIC. In certain alternative forms of the present technology, a 32-bit RISC CPU, such as an STR9 series microcontroller from ST MICROELECTRONICS or a 16-bit RISC CPU such as a processor from the MSP430 family of microcontrollers, manufactured by TEXAS INSTRUMENTS may also be suitable. In one form of the present technology, the central controller 4230 is a dedicated electronic circuit. In one form, the central controller 4230 is an application-specific integrated circuit. In another form, the central controller 4230 comprises discrete electronic components. The central controller 4230 may be configured to receive input signal(s) from one or more transducers 4270, one or more input devices 4220, and the humidifier 5000.

The central controller 4230 may be configured to provide output signal(s) to one or more of an output device 4290, a therapy device controller 4240, a data communication interface 4280, and the humidifier 5000.

In some forms of the present technology, the central controller 4230 is configured to implement the one or more methodologies described herein, such as the one or more algorithms expressed as computer programs stored in a non-transitory computer readable storage medium, on an internal memory. In some forms of the present technology, the central controller 4230 may be integrated with an RPT device 130. However, in some forms of the present technology, some methodologies may be performed by a remotely located device such as a mobile computing device. For example, the remotely located device may determine control settings for a ventilator or detect respiratory related events by analysis of stored data such as from any of the sensors described herein. As explained above, all data and operations for external sources or the central controller 4230 are generally proprietary to the manufacturer of the RPT device 130. Thus, the data from the sensors and any other additional operational data is not generally accessible by any other device.

In one form of the present technology, a data communication interface is provided, and is connected to the central controller 4230. The data communication interface may be connectable to a remote external communication network and/or a local external communication network. The remote external communication network may be connectable to remote external devices such as servers or databases. The local external communication network may be connectable to a local external device such as a mobile device or a health monitoring device. Thus, the local external communication network may be used by either the RPT device 130 or a mobile device to collect data from other devices.

In one form, the data communication interface is part of the central controller 4230. In another form, data communication interface 4280 is separate from the central controller 4230, and may comprise an integrated circuit or a processor. In one form, the remote external communication network is the Internet. The data communication interface may use wired communication (e.g. via Ethernet, or optical fiber) or a wireless protocol (e.g. CDMA, GSM, 2G, 3G, 4G/LTE, LTE Cat-M, NB-IoT, 5G New Radio, satellite, beyond 5G) to connect to the Internet. In one form, local external communication network 4284 utilizes one or more communication standards, such as Bluetooth, or a consumer infrared protocol.

The example RPT device 130 includes integrated sensors and communication electronics as shown in FIG. 3C. Older RPT devices may be retrofitted with a sensor module that may include communication electronics for transmitting collected data. Such a sensor module could be attached to the RPT device and thus transmit operational data to an external device such as the management server 110 or the user device 120.

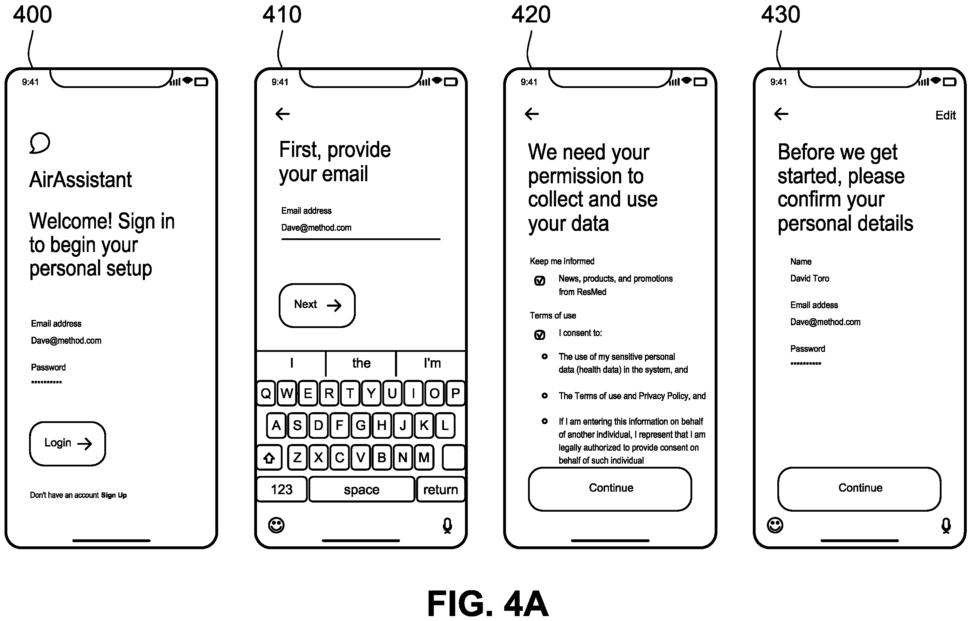

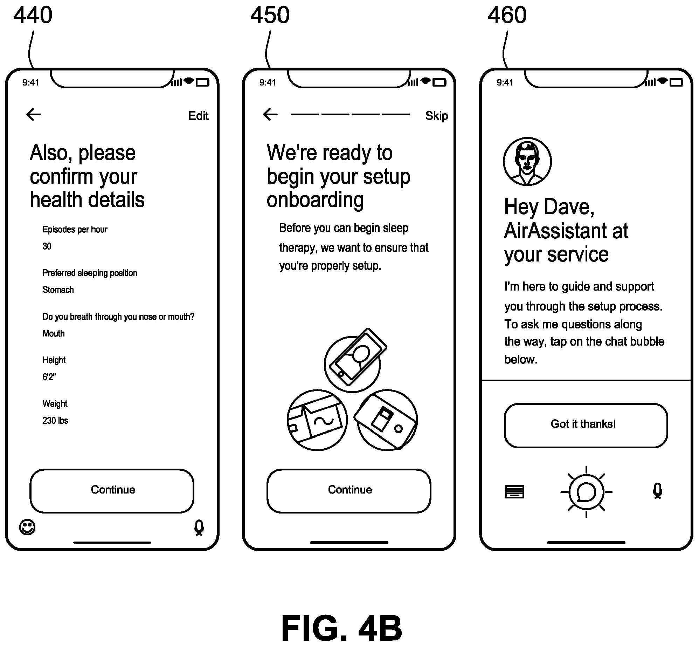

FIGs. 4A-4B illustrate screenshots provided by the client application 125 associated with an initial welcome, login, and onboarding process. FIG. 4A thus shows screenshots for user interfaces including a welcome interface 400, a login screen 410, a terms and conditions screen 420, and a personal data confirmation screen 430. FIG. 4B shows screenshots for user interfaces including a health details confirmation screen 440, an onboarding overview screen 450, and an assistant introduction screen 460. The client application 125 may present these user interfaces when the client device 120 opens the client application 125 for the first time after it is downloaded and installed. Here, the client application 125 obtains various profile information from the patient (e.g., personal information and health information) and permissions via the terms and conditions screen 420, the personal data confirmation screen 430, and the health details confirmation screen 440 to collect and use additional data from the patient. The collected information may be transmitted from the client device 120 to the management server 110 to be stored in association with the patient's user profile. The assistant introduction screen 460 allows a user to ask questions along the installation process via a chat window.

FIGs. 5A-5B illustrate screenshots provided by the client application 125 in association with an unboxing and equipment identification process. FIG. 5A therefore shows screenshots of an overview screen 500 and an instruction screen 510. Here, the client application 125 presents a user interface with step-by-step instructions for the patient to follow. After instructing the patient to unbox the equipment in the overview screen 500, the client application 125 prompts the patient to point the camera of the client device 110 at the sleep apnea device 130 via the instruction screen 510.

An example screenshot of a device image capture interface screen 530 displayed by the client activation after activation from the instruction screen 510 is shown in FIG. 5B. The device image capture interface screen 530 includes a reticle 532 that may be centered around the image of the RPT device 130. A device image processing screen 540 is displayed while the captured device image is processed. The captured image is sent to the management server 110 to enable the device recognition module 204 to automatically recognize the type of sleep apnea device 130 and store this information to the patient's profile. The client application 125 then confirms to the patient successful identification of the sleep apnea device 130. A device confirmation screen 550 is displayed with a graphical image 552 showing the model of the identified sleep apnea device is thus displayed on confirmation. Such information may be used to confirm that the assembly/set up of the product and associated peripherals/connections (power, tubing, etc.) are correct for the product which the patient will use to receive the respiratory therapy.

The client application 125 similarly prompts the patient to point the camera of the client device 110 to the mask 140 and sends the captured image to the management server 110. An example screenshot of a mask image capture interface screen 560 displayed by the client activation after activation from the instruction screen 510 is shown in FIG. 5B. The mask image capture interface screen 560 includes a reticle 562 that may be centered around the image of the mask 140. A mask image processing screen 570 is displayed while the captured mask image is processed. The mask recognition module 206 automatically recognizes the type of mask 140 based on this image and stores the mask type to the patient's profile. A mask confirmation screen 580 is displayed with a graphic image 582 showing the model of the identified mask is thus displayed on confirmation.

FIG. 5C illustrates a screenshot of an interface 590 provided by the client application 125 in association with a complete mask assembly process. Here, the client application 125 may present a series of images or a video (e.g., from the media database 252) instructing the patient on how to properly assemble the mask 140. The presentation may be specific to the particular mask type identified previously. The mask identification information may also be used to provide information about specific components, their use/function, and proper care (i.e. how to uninstall, how to clean. As indicated, this would be specific to the mask type previously identified as there are a large number of mask variations, and their composition and function can vary.

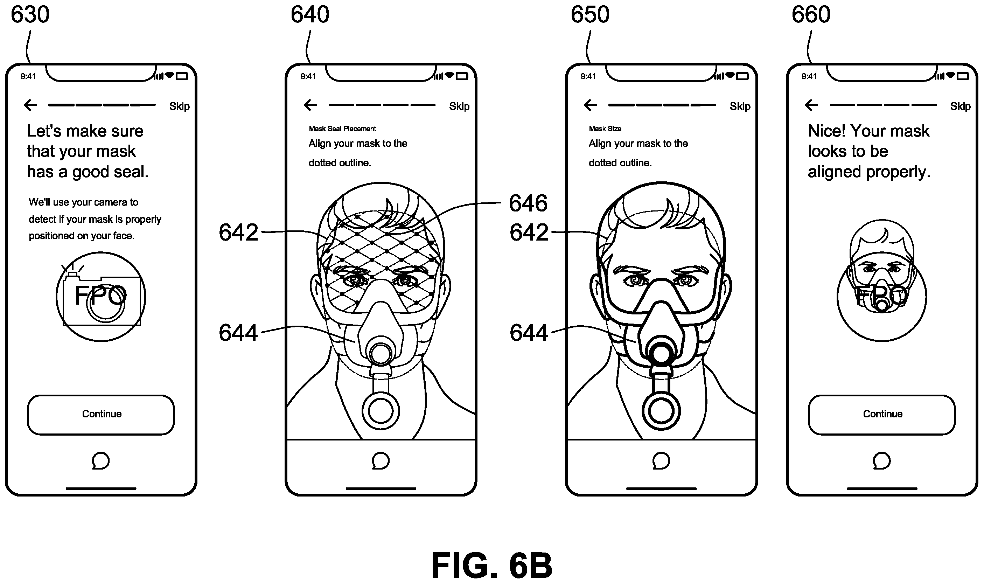

FIGs. 6A-6B illustrate screenshots provided by the client application 125 in association with assisting a patient with mask placement. FIG. 6A is a screenshot of an overview interface 600 that instructs a user to try on the mask 140. Here, the client application 125 provides step-by-step instructions (e.g., in the form of text, images, video or combination thereof) instructing the patient on how to put on the mask 140. FIG. 6B is a screenshot of an example video 610 that instructs the patient on how to put on the mask 140. Once the patient indicates that the mask 140 is being worn, the client application 125 presents an interface 620 offering to assist the patient in properly positioning the mask seal on the face to ensure that the seal is correct.

If the patient selects an option for assistance on the interface 620, the client application 125 may capture video of the user's face using a user-facing camera of the client device 110 and present an augmented reality view on the display that includes the captured video and overlays markers on the patient's face that indicate proper mask placement. FIG. 6B shows a sequence of screenshots for capturing video to assist in mask placement. An instruction overview image 630 is displayed to a patient to initiate the video capture. Using the augmented reality view, the user is instructed to align to mask 140 with the markers in order to properly position the mask 140. A selfie interface 640 shows the imposition of a mask outline 642 on a captured self-image 644 that includes the face of the patient wearing the mask 130. The mask outline 642 may include location markers 646 to assist in alignment with facial features. The mask in the self-image 644 may be adjusted as shown in a second image of a selfie interface 650 that assists the patient in adjusting the mask to match the mask outline 642.

In an embodiment, the client application 125 may detect when the mask is properly aligned based on the captured video and alert the patient. The selfie interface 650 will generate symbols such as arrows that assist the patient in moving the mask to align with the mask outline 642. The interface 650 will show the image of the actual mask 130 in relation with the mask outline 642 to assist in the correct alignment of the mask. Once the mask is correctly located, a placement confirmation interface 660 is displayed indicating successful placement to the patient.

FIGs. 7A-7C illustrate screenshots associated with a mask leak detection process that may be part of the client application 125. Once the mask 140 is detected to be properly aligned, the client application 125 may instruct the patient to hold the client device 120 near the mask 140. The client application 125 displays a leak coordination overview interface 700 to instruct the patient to detect the noise of a leak during operation of the respiratory therapy device 130. The client application 125 controls a microphone of the client device 120 to capture audio and send the audio or extracted features from the audio (e.g., audio fingerprints) to the management server 110. When the patient selects the continue button in the interface 700, a microphone interface 710 is displayed to allow a patient to activate the microphone of the client device 120. An active microphone interface 720 is displayed when the microphone is activated. The mask leak detection module 210 may then detect, based on the extracted features, if the mask 140 has a proper seal or an acceptable leak profile. In an embodiment, the detection algorithm may be different for different types of masks 140 and/or for different facial features. For example, a different detection algorithm may be used on patients with facial hair than the one used for patients without facial hair.

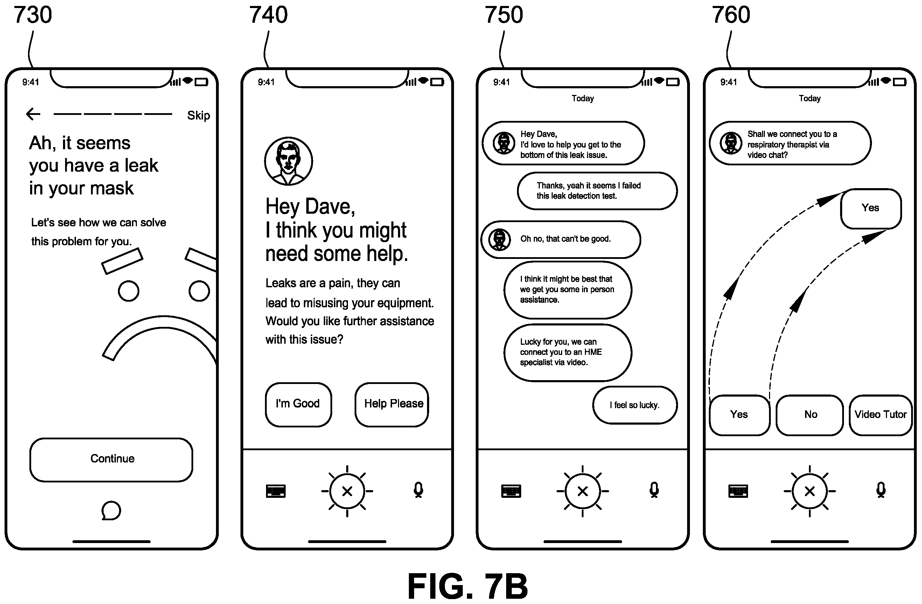

Upon detecting a proper seal, the patient may be alerted via the client application 125 that the testing is complete. Otherwise, if a proper seal is not detected, the client application 125 may alert the patient and provide the patient with the option of repositioning the mask 140 and trying again accessing the interfaces shown in FIGs. 6A-6C above. FIG. 7B shows a leak detected interface 730 that alerts the patient based on a detected leak. Alternatively, the client application 125 may provide the patient an option to obtain assistance from an automated assistant or from a live human representative. An example assistant introduction interface 740 may be activated to allow communication to an assistant. For example, in one embodiment, the patient can chat with the assistant via a chat interface 750. If the chat with the remote assistant fails to resolve the problem, the client application 125 may initiate a video call with a live representative to further assist the patient as shown in a video initiation interface 760. The video initiation interface 760 allows the display of a confirmation screen 770. If the patient confirms the need to escalate to a live representative, the application will initiate a video call screen 780 allowing the patient to initiate the video call with the live representative. If the issue cannot be resolved, the client application 125 may recommend that the patient order a different size or model of mask 140.

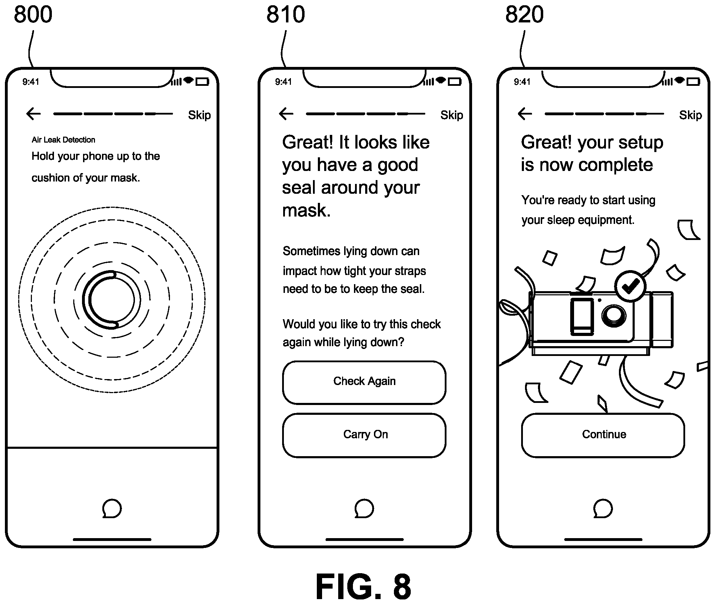

FIG. 8 illustrates screenshots provided by the client application 125 in association with a setup completion process. Here, the client application 125 presents screenshots 800, 810 and 820 informing the patient that the setup is complete and may provide the patient the option of repeating any of the earlier steps. The screenshot 800 shows an interface 800 that displays the status. The screenshot 810 is a completion interface screen that allows the patient to repeat the process or complete the process. The patient may choose to end the setup resulting in the display of the completion screen 820.

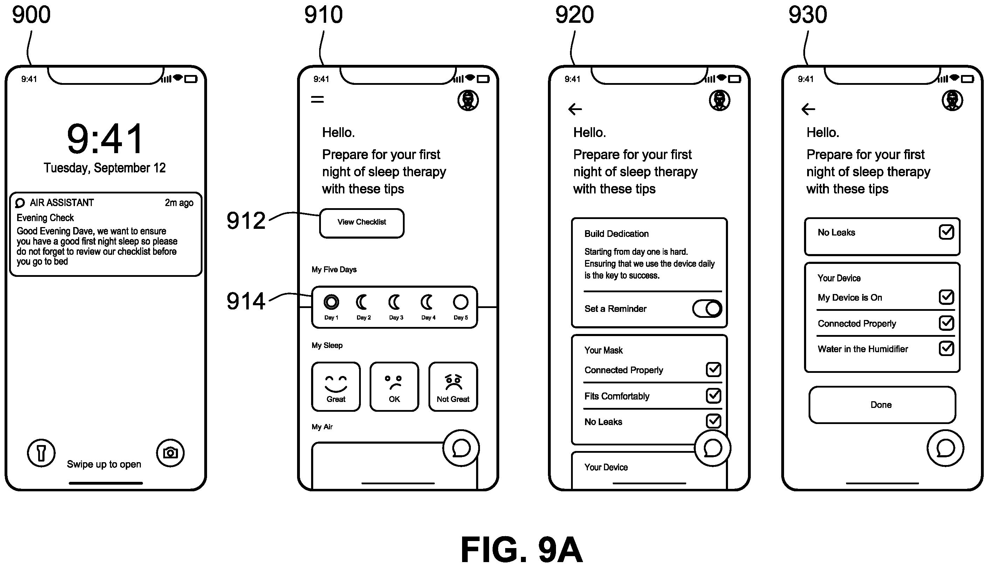

FIG. 9A illustrates screenshots provided by the client application 125 in association with preparing the patient for the first night of therapy with the newly setup sleep apnea device 130. In an embodiment, the client application 125 may present a notification user interface 900 automatically in advance of the patient's normal bed time. Here, the client application 125 may present the patient with information relevant to preparing for the first night of therapy and may present a checklist for the patient to ensure that all equipment is properly set up and ready. For example, an interface 910 may allow a user to select a checklist button 912 for information relevant to preparing for the first night of therapy. The interface 910 includes icons 914 representing the days that the sleep apnea device 130 has been used. One example checklist interface is a mask checklist screen 920 that provides checks for the mask placement such as whether the mask is connected properly, fits comfortably and there are no leaks. Another example checklist interface is a device checklist screen 930 that provides checks for device operation such as whether the device is on, whether the device is connected properly and whether there is water in the humidifier.

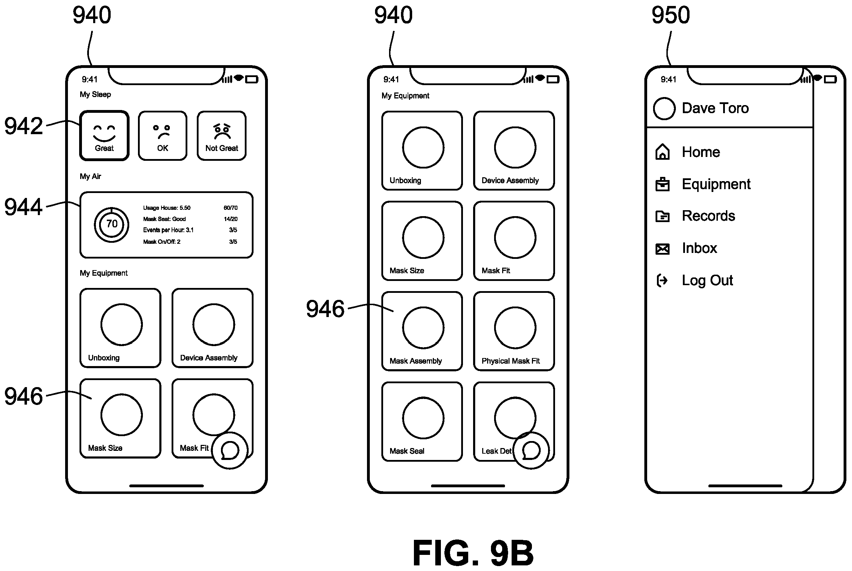

FIG. 9B shows a master interface 940 that includes a sleep input section 942 that allows a user to select icons representing quality of sleep, a data field 944 representing summary data of each therapy session using the device 130 and an overall score, and different activation buttons for functions of the client application 125. The interface 950 shows menu options that allow a patient to access different application functions.

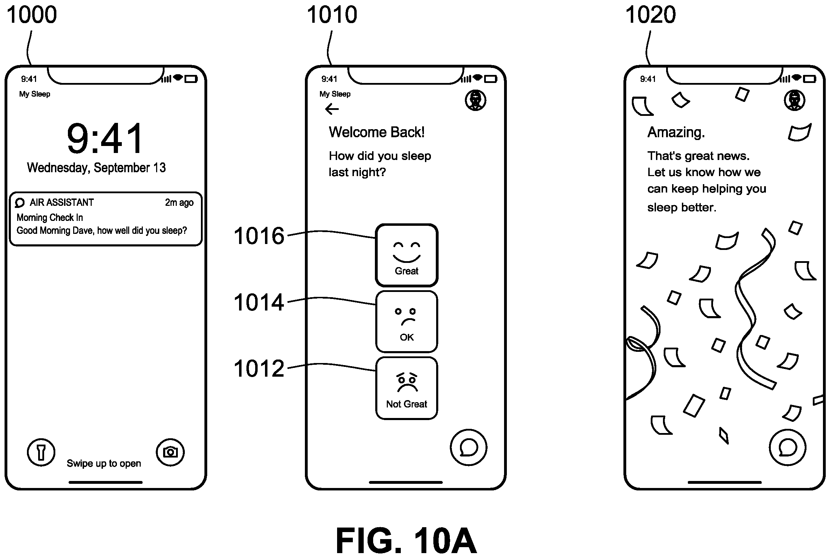

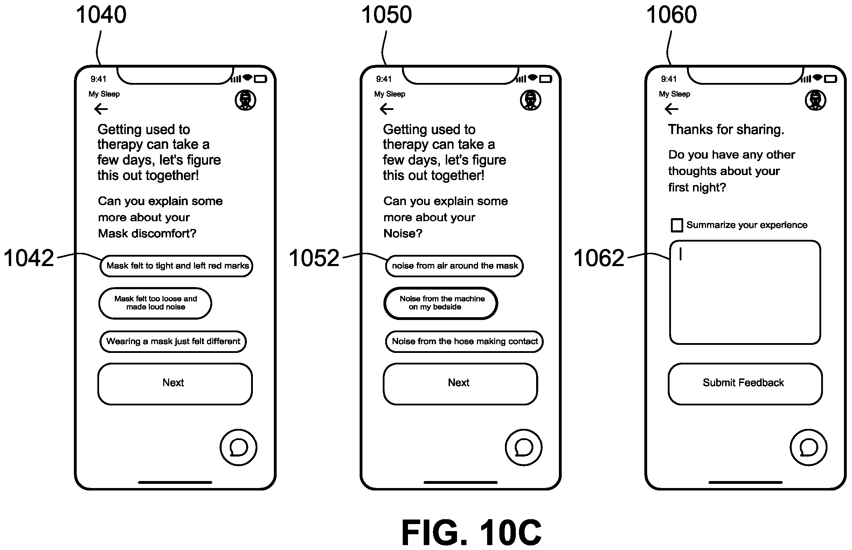

FIGs. 10A-10C illustrate screenshots provided by the client application 125 in association with obtaining feedback from the patient following the first night of therapy. Here, the client application 125 may present a notification user interface 1000 in the morning near the patient's predicted wake up time. The client application 125 may solicit feedback from the patient regarding the quality of the patient's sleep overnight. For example, a sleep rating interface 1010 may be presented that includes selectable icons 1012, 1014, and 1016 that a patient may select reflecting their sleep experience. The feedback may be sent to the management server 110. Positive feedback such as selecting the icon 1016 may allow the presentation of an additional context interface 1020 that allows additional data to be collected.

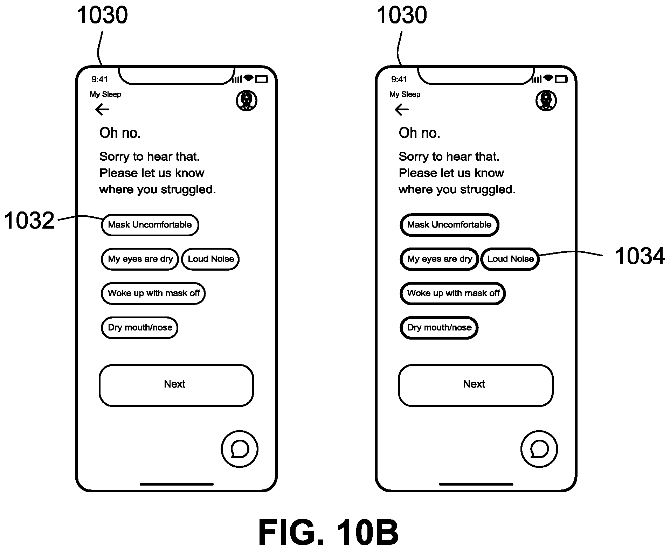

If the patient provides negative feedback such as selecting the icon 1012 or the icon 1014 in the interface 1010, the client application 125 may present additional questions relevant to diagnosing the reason for the patient's bad experience. For example, an appliance analysis feedback screen 1030 shown in FIG. 10B may present different options 1032 that allow a user to select reasons why they were dissatisfied such as the mask being uncomfortable, eyes being dry, loud noise, waking up with the mask off and a dry mouth/nose. After selection, the interface 1030 will show selected problem icons as highlighted icons 1034 and send the data to the management server 110. An example mask discomfort interface 1040 may be displayed to collect data on potential mask discomfort via icons 1042 representing common mask related complaints such as the mask felt too light, the mast felt too loose and made loud noise, or the mask felt foreign on the face. Each of the selected icons may cause other feedback interfaces to be displayed. For example, if noise is identified as a problem, a noise feedback interface 1050 may be displayed to collect data on noise issues via icons 1052 representing common noise problems such as noise from air around the mask, noise from the machine, or noise from the hose making contact. This feedback may furthermore be sent to the management server 110 and may be used to generate recommendation for the patient (or for a population of patients) to help improve the experience. An example summary and submit interface 1060 may be displayed with a comment box 1062 for the patient to provide additional feedback.

FIG. 11 illustrates an example embodiment of the mask recognition module 206. Here, the mask recognition module 206 includes a learning module 1110 and a prediction module 1120. The learning module 1110 learns correlations between image features derived from images of masks 140, mask CAD files, and the type of mask 140. In an embodiment, the learning module 1110 comprises a data acquisition module 1112, a dataset preparation module 1114, and a machine learning and evaluation module 1116. The data acquisition module 1112 acquires imaging datasets with sufficient sample size and condition variation to enable the machine learning. For example, the data acquisition module 1112 may acquire images of each possible type of mask capturing under various lighting conditions, environments, orientations, and using different acquisition devices in order to obtain a dataset with a wide range of images, representative of those that may be captured by patients during the mask detection process described above in FIGs. 5A-5B. The dataset preparation module 1114 prepares the imaging dataset for machine learning by performing various processing on the images. For example, the dataset preparation module 1114 may normalize the images, put the images into a standardized format, and perform one or more transformations on the images (e.g., to extract image features). The machine learning and evaluation module 1116 trains a machine learning model to learn correlations between the images and the mask type. In different embodiments, the machine learning and evaluation module 1116 may perform supervised learning, unsupervised learning, or a combination thereof. The machine learning and evaluation module 1116 may generate a candidate model 1118 that represents the learned correlations.

The prediction module 1120 predicts a mask type based on a received image. In an embodiment, the prediction module 1120 comprises a deploy module 1122, a field trial module 1124, and a production roll out module 1126. The deploy module 1122 transforms the candidate model 1118 to machine learning models that can be deployed across multiple machine learning platforms. The field trial module 1124 manages controlled field trials to evaluate the predictions of the candidate model 1118 under different conditions using the multiple machine learning platforms. The field trial module 1124 may refine the candidate model 1118 to generate a validated model 1128. The production roll out module 1126 applies the validated model 1128 to input images of masks received during the patient setup and generates the mask type prediction. The production roll out module 1126 may incorporate various analytics and data collection mechanisms to generate updates to the validated model 1128 to continue to improve its accuracy.

FIG. 12 illustrates an example embodiment of the mask recommendation module 212. The mask recommendation module 212 recommends a particular mask type and size for a particular patient based on a portrait image of the patient. For example, in an embodiment, the mask recommendation module 212 applies a plurality of different feature extraction modules 1202 to identify various predicted features associated with the image of the patient. For example, the feature extraction modules 1202 may include, for example, a camera, lens, and image attributes module 1220 to identify a camera type, lens type, and various image attributes associated with the input image; a facial feature analysis and measurement module 1222 to analyze and measure facial features of the patient captured in the input image; a head skew/rotation module 1224 to detect an amount of head skew and/or rotation in the input image; a gender recognition module 1226 to detect a gender of the patient from the input image; an age estimation module 1228 to estimate an age of the patient based on the input image; and an ethnicity recognition module 1230 to predict an ethnicity of the patient based on the input image.

The features from the feature extraction modules 1212 are inputted to one or more machine-learning models 1204 that are each trained to detect suitability of a patient's face to different sizes and types of masks 140. The machine-learning models 1204 generate a mask size recommendation 1206 and a mask type recommendation 1208.

In alternative embodiments, the various modules attributed to the management server 110 described above may instead be performed in whole or in part by the client application 125. For example, instead of the client application 125 sending an image or image features to the management server 110, the client application 125 may instead directly apply one or more machine-learning models (based on a model received from the management server 110). In other embodiments, functions described herein as being performed by the client application 125 may instead be performed by the management server 110.

As used in this application, the terms "component," "module," "system," or the like, generally refer to a computer-related entity, either hardware (e.g., a circuit), a combination of hardware and software, software, or an entity related to an operational machine with one or more specific functionalities. For example, a component may be, but is not limited to being, a process running on a processor (e.g., digital signal processor), a processor, an object, an executable, a thread of execution, a program, and/or a computer. By way of illustration, both an application running on a controller, as well as the controller, can be a component. One or more components may reside within a process and/or thread of execution, and a component may be localized on one computer and/or distributed between two or more computers. Further, a "device" can come in the form of specially designed hardware; generalized hardware made specialized by the execution of software thereon that enables the hardware to perform specific function; software stored on a computer-readable medium; or a combination thereof.

The terminology used herein is for the purpose of describing particular embodiments only, and is not intended to be limiting of the invention. As used herein, the singular forms "a," "an," and "the" are intended to include the plural forms as well, unless the context clearly indicates otherwise. Furthermore, to the extent that the terms "including," "includes," "having," "has," "with," or variants thereof, are used in either the detailed description and/or the claims, such terms are intended to be inclusive in a manner similar to the term "comprising."

Unless otherwise defined, all terms (including technical and scientific terms) used herein have the same meaning as commonly understood by one of ordinary skill in the art. Furthermore, terms, such as those defined in commonly used dictionaries, should be interpreted as having a meaning that is consistent with their meaning in the context of the relevant art, and will not be interpreted in an idealized or overly formal sense unless expressly so defined herein.

While various embodiments of the present invention have been described above, it should be understood that they have been presented by way of example only, and not limitation. Although the invention has been illustrated and described with respect to one or more implementations, equivalent alterations and modifications will occur or be known to others skilled in the art upon the reading and understanding of this specification and the annexed drawings. In addition, while a particular feature of the invention may have been disclosed with respect to only one of several implementations, such feature may be combined with one or more other features of the other implementations as may be desired and advantageous for any given or particular application. Thus, the breadth and scope of the present invention should not be limited by any of the above described embodiments. Rather, the scope of the invention should be defined in accordance with the following claims.

## Claims

1. A system to provide assistance to a patient for using a respiratory therapy device and a mask for treatment of respiratory ailments, the system comprising:
an equipment database storing data relating to a plurality of device types and a plurality of mask types;
a device recognition module operable to identify the type of the respiratory therapy device from an image of the respiratory therapy device captured by a client computing device in comparison with the data relating to the plurality of device types;
a mask recognition module operable to identify the type of the mask from an image of the mask captured by the client computing device in comparison with the data relating to the plurality of mask types;
a media database including media relating to assistance information relating to at least one of a mask type or a device type;
a management server operable to send media relating to assistance information for the identified type of mask or identified type of device to the client computing device; and
a mask positioning module operable to:
receive a facial image of the patient captured by the client computing device via the interface;
perform a facial analysis to identify and track locations of facial landmarks from the facial image;
overlay a mask placement image on the received facial image frames aligned to
the detected facial landmarks for display on the client computing device; and provide instructions to the client computing device on adjusting the mask based
on the overlaid mask placement image.

2. The system of claim 1, wherein the mask positioning module provides the instructions to adjust the mask with markers associated with the facial image displayed on the client computing device.

3. The system of any one of claims 1-2, further comprising a mask leak detection module operable to detect whether the mask is properly sealed around the face of the patient based on captured audio data of breathing from the client computing device while the respiratory therapy device is in operation.

4. The system of any one of claims 1-3, wherein the device recognition module includes a machine-learning model operable to identify the type of device based on learned correlations of a device image and device types.

5. The system of any one of claims 1-4, wherein the mask recognition module includes a machine-learning model operable to identify the type of mask based on learned correlations of a mask image and mask types.

6. The system of any one of claims 1-5, wherein the client computing device includes an interface to collect input data from the patient related to respiratory therapy; and wherein the collected input data is received by the management server via an interface.

7. The system of claim 6, further comprising a patient profile database including the identified device type and identified mask type correlated with the collected input data.

8. The system of any one of claims 1-7, wherein the client device is operable to instruct the user for at least one of unboxing the respiratory therapy device, setting up the respiratory therapy device, or the usage of the respiratory therapy device.

9. The system of any one of claims 1-8, wherein the respiratory therapy device is one of a continuous positive airway pressure (CPAP) device, an automatic positive airway pressure (APAP) device, a bilevel positive airway pressure device (BiPAP).

10. The system of any_one of claims 1-9, further comprising a client application executed by the client computing device, the client application including the device recognition module and the mask recognition module.

11. The system of claim 10, wherein the client application includes an interface to enable the patient to provide feedback to the management server relating to the mask or the respiratory therapy device after a respiratory treatment.

12. The system of any one of claims 1-11, wherein the device recognition module and the mask recognition module are executed by the management server.

13. A method for providing automated assistance to a patient using a respiratory therapy device connected to a mask, the method comprising:
capturing an image of the respiratory therapy device and an image of the mask by a client computing device;
identifying the type of the respiratory therapy device from an image of the respiratory therapy device captured by the client computing device in comparison with the data relating to the plurality of device types in an equipment database via a device recognition module;
identifying the type of the mask from an image of the mask captured by the client computing device in comparison with the data relating to the plurality of mask types in the equipment database via a mask recognition module;
sending media relating to assistance information for the identified type of mask or identified type of device to the client computing device via a management server; and
receiving a facial image of the patient captured by the client computing device via the interface;
performing a facial analysis to identify and track locations of facial landmarks from the facial image via a mask positioning module;
overlaying a mask placement image on the received facial image frames aligned to the
detected facial landmarks for display on the client computing device; and providing instructions to the client computing device on adjusting the mask based on
the overlaid mask placement image.

## Patentansprüche

1. System zum Bereitstellen von Unterstützung für einen Patienten bei der Verwendung einer Atemtherapievorrichtung und einer Maske zur Behandlung von Atembeschwerden, wobei das System Folgendes umfasst:
eine Gerätedatenbank, die Daten in Bezug auf eine Mehrzahl von Vorrichtungstypen und eine Mehrzahl von Maskentypen speichert;
ein Vorrichtungserkennungsmodul, das dazu ausgelegt ist, den Typ der Atemtherapievorrichtung aus einem von einer Client-Computervorrichtung aufgenommenen Bild der Atemtherapievorrichtung im Vergleich mit Daten in Bezug auf die Mehrzahl von Vorrichtungstypen zu identifizieren;
ein Maskenerkennungsmodul, das dazu ausgelegt ist, den Typ der Maske aus einem von der Client-Computervorrichtung aufgenommenen Bild der Maske im Vergleich mit den Daten in Bezug auf die Mehrzahl von Masken zu identifizieren;
eine Mediendatenbank, die Medien in Bezug auf Unterstützungsinformationen in Bezug auf mindestens einen von einem Maskentyp oder einem Vorrichtungstyp umfasst;
einen Verwaltungsserver, der dazu ausgelegt ist, Medien in Bezug auf Unterstützungsinformationen für den identifizierten Typ von Maske oder den identifizierten Typ von Vorrichtung an die Client-Computervorrichtung zu senden; und
ein Maskenpositionierungsmodul, das ausgelegt ist zum:
Empfangen eines von der Client-Computervorrichtung über die Schnittstelle aufgenommenen Gesichtsbildes des Patienten;
Durchführen einer Gesichtsanalyse, um Stellen von Gesichtslandmarken aus dem Gesichtsbild zu identifizieren und zu verfolgen;
Auflegen eines Maskenplatzierungsbildes auf die empfangenen Gesichtsbild-Frames, die mit den detektierten Gesichtslandmarken ausgerichtet sind, zur Anzeige auf der Client-Computervorrichtung; und
Bereitstellen von Anweisungen für die Client-Computervorrichtung zum Anpassen der Maske basierend auf dem aufgelegten Maskenplatzierungsbild.

2. System nach Anspruch 1, wobei das Maskenpositionierungsmodul die Anweisungen zum Anpassen der Maske mit Markierungen bereitstellt, die mit dem auf der Client-Computervorrichtung angezeigten Gesichtsbild assoziiert sind.

3. System nach einem der Ansprüche 1 bis 2, ferner umfassend ein Maskenleckerkennungsmodul, das dazu ausgelegt ist, basierend auf erfassten Audiodaten der Atmung von der Client-Computervorrichtung zu erkennen, ob die Maske um das Gesicht des Patienten ordnungsgemäß abdichtet, während die Atemtherapievorrichtung in Betrieb ist.

4. System nach einem der Ansprüche 1 bis 3, wobei das Vorrichtungserkennungsmodul ein Maschinenlernmodell umfasst, das dazu ausgelegt ist, den Typ von Vorrichtung basierend auf erlernten Korrelationen eines Vorrichtungsbildes mit Vorrichtungstypen zu identifizieren.

5. System nach einem der Ansprüche 1 bis 4, wobei das Vorrichtungserkennungsmodul ein Maschinenlernmodell umfasst, das dazu ausgelegt ist, den Typ von Maske basierend auf erlernten Korrelationen eines Maskenbildes mit Maskentypen zu identifizieren.

6. System nach einem der Ansprüche 1 bis 5, wobei de Client-Computervorrichtung eine Schnittstelle zum Sammeln von Eingabedaten vom Patienten in Bezug auf die Atemtherapie umfasst und wobei die gesammelten Eingabedaten vom Verwaltungsserver über eine Schnittstelle empfangen werden.

7. System nach Anspruch 6, ferner umfassend eine Patientenprofildatenbank, die den identifizierten Vorrichtungstyp und den identifizierten Maskentyp korreliert mit den gesammelten Eingabedaten umfasst.

8. System nach einem der Ansprüche 1 bis 7, wobei die Client-Vorrichtung dazu ausgelegt ist, den Benutzer zu mindestens einem von Auspacken der Atemtherapievorrichtung, Einrichten der Atemtherapievorrichtung oder Nutzen der Atemtherapievorrichtung anzuweisen.

9. System nach einem der Ansprüche 1 bis 8, wobei die Atemtherapievorrichtung eine von einer Vorrichtung mit kontinuierlichem positivem Atemwegsdruck (CPAP), einer Vorrichtung mit automatischem positivem Atemwegsdruck (APAP) oder einer Vorrichtung mit zweistufigem positivem Atemwegsdruck (BiPAP) ist.

10. System nach einem der Ansprüche 1 bis 9, ferner umfassend eine Client-Anwendung, die von der Client-Computervorrichtung ausgeführt wird, wobei die Client-Anwendung das Vorrichtungserkennungsmodul und das Maskenerkennungsmodul umfasst.

11. System nach Anspruch 10, wobei die Client-Anwendung eine Schnittstelle umfasst, um den Patienten zu befähigen, nach einer Atembehandlung ein Feedback in Bezug auf die Maske oder die Atemtherapievorrichtung für den Verwaltungsserver bereitzustellen.

12. System nach einem der Ansprüche 1 bis 11, wobei das Vorrichtungserkennungsmodul und das Maskenerkennungsmodul vom Verwaltungsserver ausgeführt werden.

13. Verfahren zur Bereitstellung automatischer Unterstützung für einen Patienten, der eine Atemtherapievorrichtung verwendet, die mit einer Maske verbunden ist, wobei das Verfahren Folgendes umfasst:
Aufnehmen eines Bildes der Atemtherapievorrichtung und eines Bildes der Maske durch eine Client-Computervorrichtung;
Identifizieren des Typs der Atemtherapievorrichtung aus einem von der Client-Computervorrichtung aufgenommenen Bild der Atemtherapievorrichtung im Vergleich mit Daten in Bezug auf die Mehrzahl von Vorrichtungstypen in einer Gerätedatenbank mittels eines Vorrichtungserkennungsmoduls;
Identifizieren des Typs der Maske aus einem von der Client-Computervorrichtung aufgenommenen Bild der Maske im Vergleich mit den Daten in Bezug auf die Mehrzahl von Masken in der Gerätedatenbank mittels eines Maskenerkennungsmoduls;
Senden von Medien in Bezug auf Unterstützungsinformationen für den identifizierten Typ von Maske oder den identifizierten Typ von Vorrichtung mittels eines Verwaltungsservers an die Client-Computervorrichtung; und
Empfangen eines von der Client-Computervorrichtung über die Schnittstelle aufgenommenen Gesichtsbildes des Patienten;
Durchführen einer Gesichtsanalyse mittels eines Maskenpositionierungsmoduls, um Stellen von Gesichtslandmarken aus dem Gesichtsbild zu identifizieren und zu verfolgen;
Auflegen eines Maskenplatzierungsbildes auf die empfangenen Gesichtsbild-Frames, die mit den detektierten Gesichtslandmarken ausgerichtet sind, zur Anzeige auf der Client-Computervorrichtung; und
Bereitstellen von Anweisungen für die Client-Computervorrichtung zum Anpassen der Maske basierend auf dem aufgelegten Maskenplatzierungsbild.

## Revendications

1. Système pour fournir une assistance à un patient pour l'utilisation d'un dispositif de thérapie respiratoire et d'un masque pour le traitement d'affections respiratoires, le système comprenant :
une base de données d'équipements stockant des données relatives à une pluralité de types de dispositif et à une pluralité de types de masque ;
un module de reconnaissance de dispositif pouvant fonctionner pour identifier le type du dispositif de thérapie respiratoire à partir d'une image du dispositif de thérapie respiratoire capturée par un dispositif informatique client par rapport aux données relatives à la pluralité de types de dispositif ;
un module de reconnaissance de masque pouvant fonctionner pour identifier le type du masque à partir d'une image du masque capturée par le dispositif informatique client par rapport aux données relatives à la pluralité de types de masque ;
une base de données multimédia incluant un contenu multimédia relatif à des informations d'assistance relatives à au moins l'un d'un type de masque ou d'un type de dispositif ;
un serveur de gestion pouvant fonctionner pour envoyer un contenu multimédia relatif à des informations d'assistance pour le type de masque identifié ou le type de dispositif identifié au dispositif informatique client ; et
un module de positionnement de masque pouvant fonctionner pour :
recevoir une image faciale du patient capturée par le dispositif informatique client via l'interface ;
réaliser une analyse faciale pour identifier et suivre les emplacements des repères faciaux à partir de l'image faciale ;
superposer une image de placement de masque sur les trames d'image faciale reçues alignées sur les repères faciaux détectés pour l'affichage sur le dispositif informatique client ; et
fournir des instructions au dispositif informatique client sur l'ajustement du masque sur la base de l'image de placement de masque superposée.

2. Système selon la revendication 1, dans lequel le module de positionnement de masque fournit les instructions pour ajuster le masque avec des marqueurs associés à l'image faciale affichée sur le dispositif informatique client.

3. Système selon l'une quelconque des revendications 1 et 2, comprenant en outre un module de détection de fuite de masque pouvant fonctionner pour détecter si le masque est correctement scellé autour du visage du patient sur la base de données audio capturées de respiration à partir du dispositif informatique client pendant que le dispositif de thérapie respiratoire est en fonctionnement.

4. Système selon l'une quelconque des revendications 1 à 3, dans lequel le module de reconnaissance de dispositif inclut un modèle d'apprentissage automatique pouvant fonctionner pour identifier le type de dispositif sur la base de corrélations apprises d'une image de dispositif et de types de dispositif.

5. Système selon l'une quelconque des revendications 1 à 4, dans lequel le module de reconnaissance de masque inclut un modèle d'apprentissage automatique pouvant fonctionner pour identifier le type de masque sur la base de corrélations apprises d'une image de masque et de types de masque.

6. Système selon l'une quelconque des revendications 1 à 5, dans lequel le dispositif informatique client inclut une interface pour collecter des données d'entrée du patient liées à la thérapie respiratoire ; et dans lequel les données d'entrée collectées sont reçues par le serveur de gestion via une interface.

7. Système selon la revendication 6, comprenant en outre une base de données de profils de patients incluant le type de dispositif identifié et le type de masque identifié corrélés aux données d'entrée collectées.

8. Système selon l'une quelconque des revendications 1 à 7, dans lequel le dispositif client peut fonctionner pour donner une instruction à l'utilisateur pour au moins l'un parmi le déballage du dispositif de thérapie respiratoire, l'installation du dispositif de thérapie respiratoire ou l'utilisation du dispositif de thérapie respiratoire.

9. Système selon l'une quelconque des revendications 1 à 8, dans lequel le dispositif de thérapie respiratoire est l'un d'un dispositif de ventilation en pression positive continue (CPAP), d'un dispositif de ventilation en pression positive automatique (APAP), d'un dispositif de ventilation en pression positive à deux niveaux (BiPAP).

10. Système selon l'une quelconque des revendications 1 à 9, comprenant en outre une application client exécutée par le dispositif informatique client, l'application client incluant le module de reconnaissance de dispositif et le module de reconnaissance de masque.

11. Système selon la revendication 10, dans lequel l'application client inclut une interface pour permettre au patient de fournir une rétroaction au serveur de gestion concernant le masque ou le dispositif de thérapie respiratoire après un traitement respiratoire.

12. Système selon l'une quelconque des revendications 1 à 11, dans lequel le module de reconnaissance de dispositif et le module de reconnaissance de masque sont exécutés par le serveur de gestion.

13. Procédé pour fournir une assistance automatisée à un patient en utilisant un dispositif de thérapie respiratoire relié à un masque, le procédé comprenant :
la capture d'une image du dispositif de thérapie respiratoire et d'une image du masque par un dispositif informatique client ;
l'identification du type du dispositif de thérapie respiratoire à partir d'une image du dispositif de thérapie respiratoire capturée par le dispositif informatique client par rapport aux données relatives à la pluralité de types de dispositif dans une base de données d'équipements via un module de reconnaissance de dispositif ;
l'identification du type du masque à partir d'une image du masque capturée par le dispositif informatique client par rapport aux données relatives à la pluralité de types de masque dans la base de données d'équipements via un module de reconnaissance de masque ;
l'envoi d'un contenu multimédia relatif à des informations d'assistance pour le type de masque identifié ou le type de dispositif identifié au dispositif informatique client via un serveur de gestion ; et
la réception d'une image faciale du patient capturée par le dispositif informatique client via l'interface ;
la réalisation d'une analyse faciale pour identifier et suivre les emplacements des repères faciaux à partir de l'image faciale via un module de positionnement de masque ;
la superposition d'une image de placement de masque sur les trames d'image faciale reçues alignées sur les repères faciaux détectés pour l'affichage sur le dispositif informatique client ; et
la fourniture d'instructions au dispositif informatique client sur l'ajustement du masque sur la base de l'image de placement de masque superposée.
